# EUROPEAN PATENT APPLICATION

(11) **EP 4 830 743 A1**
(43) Date of publication of application: **28.10.2026**
(21) Application number: 26167586.2
(22) Date of filing: 25.03.2026
(51) Int. Cl.: A61M 1/00

(54) **NASAL SUCTION TIP AND NASAL ASPIRATOR**

(30) Priority: 22.04.2025 CN 202520772095 U
(71) Applicant: Shenzhen Lute Jiacheng Supply Chain Management Co., Ltd., Shenzhen, Guangdong 518000 (CN)
(72) Inventor: LIU, Liu, Shenzhen, 518000 (CN); LI, Siyu, Shenzhen, 518000 (CN); LUO, Chao, Shenzhen, 518000 (CN); JIA, Caihu, Shenzhen, 518000 (CN)
(74) Representative: Neves, Ana

(57) **Abstract**

Disclosed are a nasal suction tip (10) and a nasal aspirator (100). The nasal suction tip (10) is configured to communicate with the host (30) of the nasal aspirator (100), and the nasal suction tip (10) includes a main body (1), a collection chamber (2) and a nasal suction nozzle (3). The main body (1) is provided with an accommodation cavity, and the accommodation cavity is provided with a first opening. The collection chamber (2) is provided with a collection cavity (21), and the collection cavity (21) is provided with a second opening (211) and is communicated with the host (30). The collection chamber (2) is detachably accommodated in the accommodation cavity through the first opening. The nasal suction nozzle (3) is provided with a nasal suction channel (31) communicated with the collection cavity (21).

## Description

### TECHNICAL FIELD

The present application relates to the technical field of medical devices, and in particular to a nasal suction tip and a nasal aspirator.

### BACKGROUND

The nasal aspirator mainly includes a host and a nasal suction tip. The host mainly includes the air pump, the battery, the speaker, the display screen and other devices, and the nasal suction tip includes the nasal suction chamber and the soft-gel nasal suction nozzle. The host is connected to the nasal suction tip by a hose. When in use, the soft-gel nasal suction nozzle is inserted into the nasal cavity of the infant, and the air pump is started to suck out the liquid or foreign matter in the nasal cavity of the infant and temporarily store it in the nasal suction chamber, so as to unblock and relieve the discomfort caused by the blockage in the nasal cavity.

The nasal suction chamber of the nasal suction tip in the existing nasal aspirator is integrated in the nasal suction tip, and the nasal suction chamber is a single-layer structure. When cleaning the nasal suction chamber, the nasal suction tip needs to be cleaned as a whole, which easily causes water to enter other parts of the nasal suction tip, affecting the service life of the nasal suction tip.

### SUMMARY

The main purpose of the present application is to provide a nasal suction tip and a nasal aspirator, aiming to make the collection chamber an independent collection chamber body, which is convenient for separate cleaning or replacement.

To achieve the above purpose, the nasal suction tip provided in the present application is configured to communicate with the host of the nasal aspirator, and the nasal suction tip includes a main body, a collection chamber, and a nasal suction nozzle. The main body is provided with an accommodation cavity, and the accommodation cavity is provided with a first opening; the collection chamber provided with a collection cavity, the collection cavity is provided with a second opening and is communicated with the host, and the collection chamber is detachably accommodated in the accommodation cavity through the first opening; and the nasal suction nozzle is provided with a nasal suction channel communicated with the collection cavity, and the nasal suction nozzle is configured to cover and connect to an edge of the second opening.

In an embodiment, the accommodation cavity is provided with a suction tube communicated with the accommodation cavity, and an airflow channel is formed between the collection chamber and a wall of the accommodation cavity;
an air outlet is provided at the collection cavity, and the air outlet, the airflow channel and the suction tube are communicated; and
the air outlet is provided at a side wall of the collection cavity, and the air outlet is close to the second opening in a vertical direction.

In an embodiment, a recessed groove with one end open is provided at an outer wall of the collection chamber, and the recessed groove and the wall of the accommodation cavity are enclosed to form the airflow channel; and
one end of the recessed groove is communicated with the suction tube, and the air outlet is provided at a bottom of the recessed groove and is communicated with the airflow channel.

In an embodiment, the collection chamber includes a chamber body and a chamber cover, the chamber body and the chamber cover are detachably connected and enclosed to form the collection cavity, and the second opening is provided at the chamber cover.

In an embodiment, the chamber cover and the chamber body are plug-connected, and the chamber cover and an inner wall of the accommodation cavity are plug-connected.

In an embodiment, a seal member is provided between the chamber cover and the wall of the accommodation cavity.

In an embodiment, the nasal suction tip further includes a flow guide tube, wherein one end of the flow guide tube is connected to the edge of the second opening and communicated with the nasal suction channel, and the other end of the flow guide tube is located in the collection cavity.

In an embodiment, a height of the other end of the flow guide tube in a vertical direction is lower than a height of the air outlet in the vertical direction.

In an embodiment, the main body further includes a water tank spaced apart from the accommodation cavity and a control assembly provided at the main body; and
an atomization port is provided at a side wall of the water tank, the atomization port is provided with an atomization assembly, and the control assembly is electrically connected to the atomization assembly.

The present application also provides a nasal aspirator, including:
the nasal suction tip;
a host provided with an air pump; and
a connection tube, one end of the connection tube is communicated with the air pump, and the other end of the connection tube is communicated with a suction tube of the nasal suction tip.

The nasal suction tip provided in the technical solution of the present application includes a main body, a collection chamber and a nasal suction nozzle. The main body is provided with an accommodation cavity with a first opening. The collection chamber is detachably inserted into the accommodation cavity via the first opening. When the collection chamber is inserted into the accommodation cavity, the collection cavity in the collection chamber is communicated with the host, so that the host applies negative pressure to the collection cavity, generating suction to suck out foreign matter in the nasal cavity. The collection chamber is provided with a second opening, the nasal suction nozzle is connected to the second opening, and a nasal suction channel communicated with the collection cavity is formed in the nasal suction nozzle. When in use, the gas flow direction of the nasal suction tip is from the nasal suction channel, the collection cavity to the host, and foreign matter falls into the bottom of the collection chamber through the nasal suction channel for storage. The collection chamber can be removed from the accommodation cavity of the main body separately, and cleaned or replaced separately, thereby improving the service life of the nasal suction tip.

### BRIEF DESCRIPTION OF THE DRAWINGS

In order to illustrate the technical solutions in the embodiments of the present application or in the related art more clearly, the following briefly introduces the accompanying drawings required for the description of the embodiments or the related art. Obviously, the drawings in the following description are only part of embodiments of the present application. For those skilled in the art, other drawings can also be obtained according to the structures shown in these drawings without any creative effort.
FIG. 1 is a schematic structural diagram of a nasal suction tip according to an embodiment of the present application.
FIG. 2 is a cross-sectional diagram of the nasal suction tip according to an embodiment of the present application.
FIG. 3 is a schematic structural diagram of a nasal aspirator according to an embodiment of the present application.

### Description of reference signs:

100. nasal aspirator; 10. nasal suction tip; 1. main body; 11. suction tube; 12. water tank; 121. atomization port; 122. atomization assembly; 13. control assembly; 2. collection chamber; 21. collection cavity; 211. second opening; 212. air outlet; 22. airflow channel; 23. chamber body; 24. chamber cover; 25. seal member; 3. nasal suction nozzle; 31. nasal suction channel; 4. flow guide tube; 20. connection tube; 30. host.

The realization of the objective, functional characteristics, and advantages of the present application are further described with reference to the accompanying drawings.

### DETAILED DESCRIPTION OF THE EMBODIMENTS

The technical solutions of the embodiments of the present application will be described in more detail below with reference to the accompanying drawings. It is obvious that the embodiments to be described are only some rather than all of the embodiments of the present application. All other embodiments obtained by those skilled in the art based on the embodiments of the present application without creative efforts shall fall within the scope of the present application.

It should be noted that if there are directional indications, such as up, down, left, right, front, back, etc., involved in the embodiments of the present application, the directional indications are only used to explain the relative position relationship, movement status, etc. between the components in a certain specific posture. If the specific posture changes, the directional indication also changes accordingly.

In addition, if there are descriptions related to "first", "second", etc. in the embodiments of the present application, the descriptions of "first", "second", etc. are only for the purpose of description, and should not be construed as indicating or implying relative importance or implicitly indicating the number of technical features indicated. Thus, a feature delimited with "first", "second" may expressly or implicitly include at least one of that feature. Besides, the meaning of "and/or" or "or/and" appearing in the application includes three parallel scenarios. For example, "A and/or B" includes only A, or only B, or both A and B. In addition, the technical solutions between the various embodiments can be combined with each other, but must be based on the realization by those skilled in the art. When the combination of technical solutions is contradictory or cannot be realized, it should be considered that the combination of such technical solutions does not exist or falls within the scope of the present application.

The nasal aspirator mainly includes the host and the nasal suction tip. The host mainly includes the air pump, the battery, the speaker, the display screen and other devices, and the nasal suction tip includes the nasal suction chamber and the soft rubber nasal suction nozzle. The host part is connected to the nasal suction tip by a hose. When in use, the soft rubber nasal suction nozzle is inserted into the nasal cavity of the infant, the air pump is started, and the liquid or foreign matter in the nasal cavity of the infant is sucked out and temporarily stored in the nasal suction chamber, so as to unblock and relieve the discomfort caused by the blockage in the nasal cavity.

The nasal suction chamber of the nasal suction tip in the existing nasal aspirator is integrated in the nasal suction tip, and the nasal suction chamber is a single-layer structure. When cleaning the nasal suction chamber, the nasal suction tip needs to be cleaned as a whole, which is easy to cause water to enter other parts of the nasal suction tip, affecting the service life of the nasal suction tip.

The present application provides a nasal suction tip and a nasal aspirator, aiming to make the collection chamber an independent storage chamber body, which is convenient for separate cleaning or replacement.

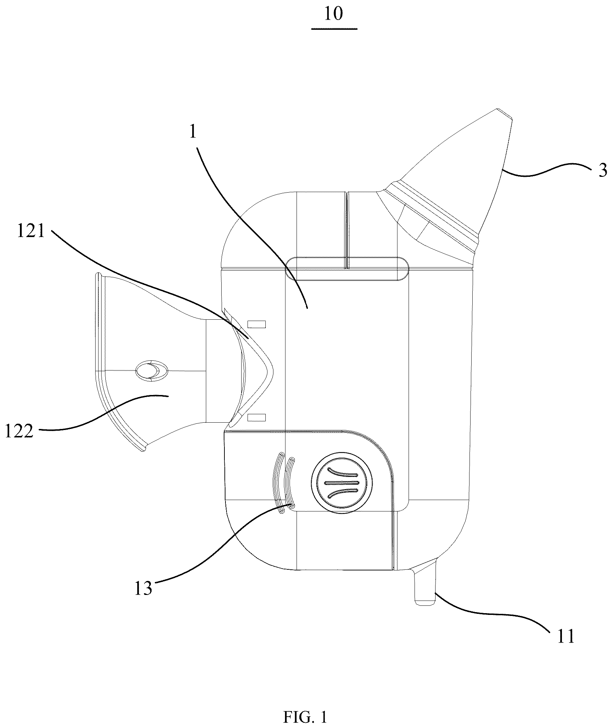

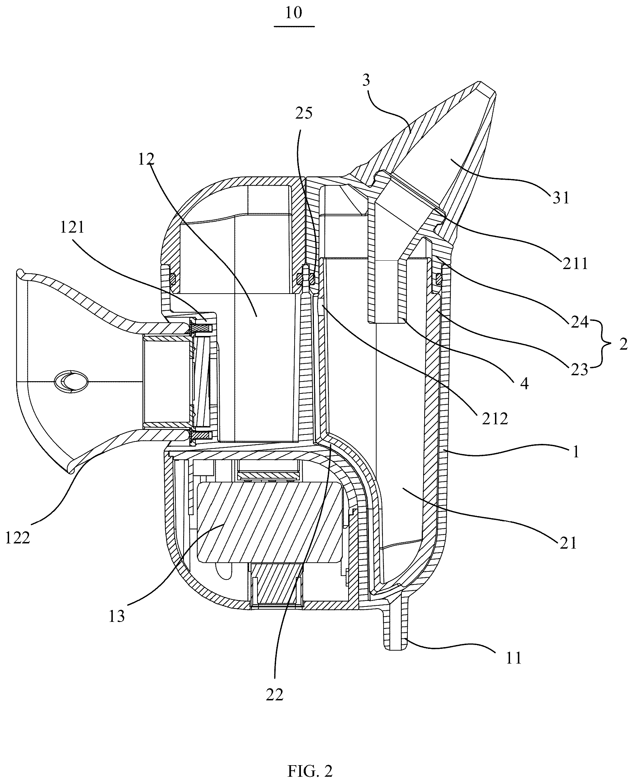

Please refer to FIG. 1 and FIG. 2, in an embodiment of the present application, the nasal suction tip 10 is configured to communicate with the host 30 of the nasal aspirator 100. The nasal suction tip 10 includes a main body 1, a collection chamber 2 and a nasal suction nozzle 3. The main body 1 is provided with an accommodation cavity with a first opening, and the collection chamber 2 is provided with a collection cavity 21 with a second opening 211. The collection cavity 21 is communicated with the host 30, and the collection chamber 2 is detachably accommodated in the accommodation cavity via the first opening. The nasal suction nozzle 3 covers the edge connected to the second opening 211, and the nasal suction nozzle 3 is provided with a nasal suction channel 31 communicated with the collection cavity 21.

In this embodiment, both the main body 1 and the collection chamber 2 are made of hard plastic material, which is relatively light and has a certain strength. Lightweight metal materials such as aluminum alloy can also be used. The nasal suction nozzle 3 is made of skin-friendly soft rubber material, which has a certain ability to deform and is non-toxic and harmless, such as medical silica gel or medical rubber. The collection chamber 2 can be inserted into the accommodation cavity of the main body 1 as a whole through the first opening, and the collection chamber 2 can be removed from the accommodation cavity as a whole for separate cleaning and replacement to prevent water from entering other parts of the main body 1 and causing failure. The relationship between the nasal suction tip 10 and the host 30 can be a split setting, where the main body 1 is communicated with the collection cavity 21 of the nasal suction tip 10 through the connection tube 20; or can be an integrated setting, where the host 30 is provided inside the accommodation cavity of the main body 1 of the nasal suction tip 10, and is directly communicated with the collection chamber 2 through a pipeline to apply negative pressure to the collection chamber 2, so as to generate suction to suck out the foreign matter in the nasal cavity.

The nasal suction tip 10 provided in the technical solution of the present application includes a main body 1, a collection chamber 2 and a nasal suction nozzle 3. The main body 1 is provided with an accommodation cavity with a first opening, a suction tube 11 communicated with the accommodation cavity is provided at the bottom of the accommodation cavity, and the collection chamber 2 is detachably inserted in the accommodation cavity via the first opening. When the collection chamber 2 is inserted in the accommodation cavity, an airflow channel 22 is formed between the collection chamber 2 and the accommodation cavity. An air outlet 212 is provided at the wall of the collection chamber 2. The air outlet 212, the airflow channel 22 and the suction tube 11 are communicated. A second opening 211 is provided in the collection chamber 2, the nasal suction nozzle 3 is connected to the second opening 211, and a nasal suction channel 31 communicated with the collection cavity 21 is formed in the nasal suction nozzle 3. When in use, the gas flow direction of the nasal suction tip 10 is from the nasal suction channel 31, the collection cavity 21, the air outlet 212, the airflow channel 22 to the suction tube 11, and the foreign matter falls into the bottom of the collection chamber 2 through the nasal suction channel 31 for storage. The collection chamber 2 can be removed from the accommodation cavity of the main body 1 separately, and cleaned or replaced separately, thereby improving the service life of the nasal suction tip 10.

In an embodiment of the present application, the accommodation cavity is provided with a suction tube 11 communicate with the accommodation cavity, an airflow channel 22 is formed between the collection chamber 2 and the wall of the accommodation cavity, and the collection cavity 21 is provided with an air outlet 212. The air outlet 212, the airflow channel 22 and the suction tube 11 are communicated. The air outlet 212 is provided at the side wall of the collection cavity 21, and the air outlet 212 is close to the second opening 211 in the vertical direction.

In this embodiment, the host 30 of the nasal aspirator 100 is communicated with the collection cavity 21 in the nasal suction tip 10 through the suction tube 11 communicated with the accommodation cavity. The airflow channel 22 may be formed through the gap between the collection chamber 2 and the accommodation cavity, or an air guide groove may be provided at the outer wall of the collection chamber 2 or the wall of the accommodation cavity. The air outlet 212 is communicated with the suction tube 11 through the air guide groove or the gap. The air outlet 212 is provided at the side wall of the collection cavity 21, and the air outlet 212 is close to the second opening 211 in the vertical direction, that is, the position of the air outlet 212 in the vertical direction is as high as possible, which can reduce the risk of foreign matter overflowing or foreign matter being sucked into the pump body through the air outlet 212 and the suction tube 11 when used in an inclined state.

In an embodiment of the present application, the outer wall of the collection chamber 2 is provided with a recessed groove with one end open, and the recessed groove and the wall of the accommodation cavity are enclosed to form an airflow channel 22. One end of the recessed groove is communicated with the suction tube 11, an air outlet 212 is provided at the bottom of the recessed groove, and the air outlet 212 is communicated with the airflow channel 22.

In this embodiment, the recessed groove provided at the collection chamber 2 and the wall of the accommodation cavity are enclosed to form the airflow channel 22 between the collection chamber 2 and the main body 1. One end of the recessed groove is communicated with the suction tube 11, and the air outlet 212 is provided at the bottom of the recessed groove. When the collection chamber 2 is installed in the accommodation cavity of the main body 1, the air outlet 212, the recessed groove and the suction tube 11 are communicated, so that the gas can pass through the nasal suction channel 31 of the nasal suction nozzle 3 and the collection cavity 21, and flow into the pump body of the host 30, so as to achieve the effect of sucking foreign matter in the nasal cavity of the infant. It can be understood that in an embodiment, the recessed groove can also be provided at the wall of the accommodation cavity, and enclosed with the outer wall of the collection chamber 2 to form the airflow channel 22, which can also achieve the effect of allowing the gas to pass through.

In an embodiment of the present application, the collection chamber 2 includes a chamber body 23 and a chamber cover 24. The chamber body 23 is detachably connected to and enclosed with the chamber cover 24 to form the collection cavity 21, and the second opening 211 is provided in the chamber cover 24.

In this embodiment, the collection chamber 2 includes a chamber body 23 and a chamber cover 24 detachably connected to the chamber body 23, and the chamber cover 24 is easy to be removed from the chamber body 23, which is easy to clean or reinstall, thereby improving the convenience of use. The detachable connection between the chamber body 23 and the chamber cover 24 can be plug-in connection, or the chamber cover 24 and the chamber body 23 can also be screwed and connected, which is not further limited here. The second opening 211 is provided at the chamber cover 24, and the nasal suction nozzle 3 is connected to the edge of the second opening 211 and is communicated with the collection cavity 21 enclosed by the chamber body 23 and the chamber cover 24. In an embodiment, the axis direction of the second opening 211 is at an angle with the axis direction of the collection cavity 21, so that the nasal suction nozzle 3 can be inserted into the nasal cavity of the infant without tilting the nasal suction tip 10 as a whole, thereby improving the convenience of using the nasal suction tip 10.

In an embodiment of the present application, the chamber cover 24 and the chamber body 23 are plug-connected, and the chamber cover 24 and the inner wall of the accommodation cavity are plug-connected.

In this embodiment, when the collection chamber 2 is assembled, the chamber cover 24 and the chamber body 23 are plug-connected, the opening edge of the chamber body 23 is plug-connected into the inner edge of the chamber cover 24, and the collection chamber 2 is inserted into the accommodation cavity of the main body 1 through the first opening. The outer periphery of the chamber cover 24 and the inner wall of the accommodation cavity are plug-connected. The plug-connection method is more convenient for disassembly, which improves the convenience of using the nasal suction tip 10.

In an embodiment of the present application, a seal member 25 is provided between the chamber cover 24 and the wall of the accommodation cavity.

In an embodiment, the seal member 25 is provided between the chamber cover 24 and the wall of the accommodation cavity. The seal member 25 can be a seal ring or a seal strip, etc. The seal member 25 can not only prevent the gas from escaping from between the chamber cover 24 and the wall of the accommodation cavity, but also play a certain fixing and limiting effect to prevent the chamber cover 24 from deflecting from the main body 1.

In an embodiment of the present application, the nasal suction tip 10 also includes a flow guide tube 4. One end of the flow guide tube 4 is connected to the edge of the second opening 211 and is communicated with the nasal suction channel 31, and the other end of the flow guide tube 4 is located in the collection cavity 21.

In this embodiment, the nasal suction tip 10 is further provided with a flow guide tube 4. One end of the flow guide tube 4 is connected to the edge of the second opening 211. One end of the flow guide tube 4 is communicated with the nasal suction channel 31 of the nasal suction nozzle 3, and the other end of the flow guide tube 4 is located in the collection cavity 21 and communicated with the collection cavity 21. The flow guide tube 4 can guide foreign matter to fall into the bottom of the collection chamber 2, and prevent the foreign matter from adhering to the side wall or the position of the second opening 211, which otherwise may affect subsequent use.

In an embodiment of the present application, the height of the other end of the flow guide tube 4 in the vertical direction is lower than the height of the air outlet 212 in the vertical direction.

In this embodiment, in the vertical direction, the height of the other end of the flow guide tube 4 is lower than the height of the air outlet 212, so that the foreign matter falls and is stored at the bottom of the collection chamber 2 through the guiding effect of the flow guide tube 4, and the gas can be sucked out through the air outlet 212, reducing the occurrence of the problem that the foreign matter is attracted by the air flow and overflows from the air outlet 212, and improving the reliability of the use of the nasal suction tip 10.

In an embodiment of the present application, the main body 1 also includes a water tank 12 spaced apart from the accommodation cavity and a control assembly 13 provided at the main body 1, and the side wall of the water tank 12 is provided with an atomization port 121. The atomization port 121 is provided with an atomization assembly 122, and the control assembly 13 is electrically connected to the atomization assembly 122.

In this embodiment, the main body 1 further includes a water tank 12 separated from the accommodation cavity, and an atomization port 121 is provided at the side wall of the water tank 12. An atomization assembly 122 is provided at the atomization port 121, and the atomization assembly 122 includes an atomization sheet and an atomization cover, etc. The liquid such as pure water or physiological saline is added to the atomization water tank 12, atomized by the atomization sheet and sprayed to the nasal cavity through the atomization cover, so as to soften the lumpy foreign matter in the nasal cavity. After softening, the foreign matter can be easily sucked out through one side of the nasal suction nozzle 3, unblocking and relieving the discomfort of the blockage in the nasal cavity. The main body 1 is also provided with a control assembly 13 electrically connected to the atomization assembly 122, and the control assembly 13 controls the start and stop of the atomization assembly 122.

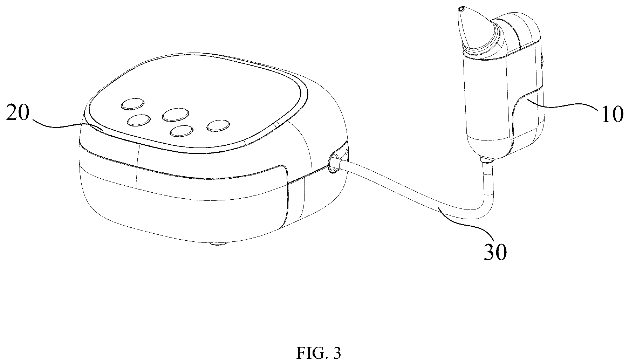

Please refer to FIG. 3. the present application also provides a nasal aspirator 100, including the nasal suction tip 10, the connection tube 20 and the host 30 as described above. The host 30 is provided with an air pump. One end of the connection tube 20 is communicated with the air pump, and the other end of the connection tube 20 is communicated with the suction tube 11 of the nasal suction tip 10.

In this embodiment, the air pump is provided in the host 30, which is communicated with the suction tube 11 of the nasal suction tip 10 through the connection tube 20. The host 30 is also provided with a control button electrically connected to the air pump. When the air pump is started, gas enters the air pump of the host 30 through the nasal suction channel 31 of the nasal suction nozzle 3, the collection cavity 21, the air outlet 212, the airflow channel 22 and the suction tube 11, thereby achieving the effect of sucking out foreign matter in the nasal cavity of the infant, and the foreign matter directly falls into the bottom of the collection chamber 2.

The above are only embodiments of the present application, and do not limit the scope of the present application. Under the technical concept of the present application, all equivalent structural changes made by using the contents of the specification and drawings of the present application, or directly/indirectly applied in other related technical fields, are included in the scope of the present application.

## Claims

1. A nasal suction tip (10), configured for communicating with a host (30) of a nasal aspirator (100), **characterized by** comprising:
a main body (1) provided with an accommodation cavity, wherein the accommodation cavity is provided with a first opening;
a collection chamber (2) provided with a collection cavity (21), wherein the collection cavity (21) is provided with a second opening (211) and is communicated with the host (30), and the collection chamber (2) is detachably accommodated in the accommodation cavity through the first opening; and
a nasal suction nozzle (3) provided with a nasal suction channel (31) communicated with the collection cavity (21), wherein the nasal suction nozzle (3) is configured to cover and connect to an edge of the second opening (211).

2. The nasal suction tip (10) according to claim 1, wherein:
the accommodation cavity is provided with a suction tube (11) communicated with the accommodation cavity, and an airflow channel (22) is formed between the collection chamber (2) and a wall of the accommodation cavity;
an air outlet (212) is provided at the collection cavity (21), and the air outlet (212), the airflow channel (22) and the suction tube (11) are communicated; and
the air outlet (212) is provided at a side wall of the collection cavity (21), and the air outlet (212) is close to the second opening (211) in a vertical direction.

3. The nasal suction tip (10) according to claim 2, wherein:
a recessed groove with one end open is provided at an outer wall of the collection chamber (2), and the recessed groove and the wall of the accommodation cavity are enclosed to form the airflow channel (22); and
one end of the recessed groove is communicated with the suction tube (11), and the air outlet (212) is provided at a bottom of the recessed groove and is communicated with the airflow channel (22).

4. The nasal suction tip (10) according to claim 2, wherein the collection chamber (2) comprises a chamber body (23) and a chamber cover (24), the chamber body (23) and the chamber cover (24) are detachably connected and enclosed to form the collection cavity (21), and the second opening (211) is provided at the chamber cover (24).

5. The nasal suction tip (10) according to claim 4, wherein the chamber cover (24) and the chamber body (23) are plug-connected, and the chamber cover (24) and an inner wall of the accommodation cavity are plug-connected.

6. The nasal suction tip (10) according to claim 5, wherein a seal member (25) is provided between the chamber cover (24) and the wall of the accommodation cavity.

7. The nasal suction tip (10) according to any one of claims 1 to 6, further comprising a flow guide tube (4), wherein one end of the flow guide tube (4) is connected to the edge of the second opening (211) and communicated with the nasal suction channel (31), and the other end of the flow guide tube (4) is located in the collection cavity (21).

8. The nasal suction tip (10) according to claim 7, wherein a height of the other end of the flow guide tube (4) in a vertical direction is lower than a height of the air outlet (212) in the vertical direction.

9. The nasal suction tip (10) according to any one of claims 1 to 6, wherein the main body (1) further comprises a water tank (12) spaced apart from the accommodation cavity and a control assembly (13) provided at the main body (1); and
an atomization port (121) is provided at a side wall of the water tank (12), the atomization port (121) is provided with an atomization assembly (122), and the control assembly (13) is electrically connected to the atomization assembly (122).

10. A nasal aspirator (100), **characterized by** comprising:
the nasal suction tip (10) according to any one of claims 1 to 9;
a host (30) provided with an air pump; and
a connection tube (20), wherein one end of the connection tube (20) is communicated with the air pump, and the other end of the connection tube (20) is communicated with a suction tube (11) of the nasal suction tip (10).
